# EUROPÄISCHE PATENTSCHRIFT

(11) **EP 4 138 717 B1**
(45) Veröffentlichungstag und Bekanntmachung des Hinweises auf die Patenterteilung: **28.05.2025**
(21) Anmeldenummer: 21720252.2
(22) Anmeldetag: 20.04.2021
(51) Int. Cl.: A61B 90/98, A61B 17/16, A61B 90/00

(54) **MEDIZINISCHES WERKZEUG MIT ENTKUPPLUNGSERKENNUNG**
MEDICAL TOOL HAVING DECOUPLING RECOGNITION
INSTRUMENT MÉDICAL À RECONNAISSANCE DE DÉSACCOUPLEMENT

(30) Priorität: 22.04.2020 DE 102020110918
(43) Veröffentlichungstag der Anmeldung: 01.03.2023
(73) Patentinhaber: Aesculap AG, 78532 Tuttlingen (DE)
(72) Erfinder: LENZENHUBER, Frederick, 78532 Tuttlingen (DE)
(74) Vertreter: Winter, Brandl - Partnerschaft mbB
(86) Internationale Anmeldenummer: PCT/EP2021/060180
(87) Internationale Veröffentlichungsnummer: WO 2021/214026

(56) Entgegenhaltungen:
- EP-A1- 2 581 061
- WO-A2-03/013372
- WO-A2-2009/138242
- DE-B1- 2 916 221
- US-A1- 2009 065 565
- US-A1- 2018 256 287

## Beschreibung

### Technisches Gebiet

Die vorliegende Offenbarung betrifft ein medizinisches Werkzeug, das als ein drehantreibbares Werkzeug, insbesondere als ein spanabhebendes Werkzeug, vorzugsweise als ein Trepanationswerkzeug, ausgebildet ist und das einen mit einer medizinischen Vorrichtung, vorzugsweise einem chirurgisches Handstück, zur insbesondere formschlüssigen Drehmomentübertragung verbindbaren Antriebsabschnitt und einen mit dem Antriebsabschnitt drehmomentübertragend koppelbaren Schneidabschnitt hat.

Trepanationswerkzeuge werden eingesetzt, um eine operative Öffnung im Schädelknochen einzubringen. Um Beschädigungen an der unter dem Schädelknochen angeordneten Dura auszuschließen, sind Trepanationswerkzeuge funktional so aufgebaut, dass der Schneidabschnitt von dem eigentlichen Antrieb, also dem Antriebsabschnitt, abgekoppelt wird, sobald der Schädelknochen durchdrungen ist und bevor die unter dem Schädelknochen liegende Dura beschädigt werden kann. Dies wird üblicherweise, wie beispielsweise in der US 4,456,010 A1, und bei einem gattungsgemäßen Werkzeug dadurch erreicht, dass der Schneidabschnitt relativ zu dem Antriebsabschnitt axial begrenzt zwischen einer ersten Axialposition, in der der Schneidabschnitt und der Antriebsabschnitt drehmomentgekoppelt sind, und einer zweiten Axialposition, in der der Schneidabschnitt von dem Antriebsabschnitt drehmomententkoppelt ist, verlagerbar ist. Im Zerspanungseingriff wird der Schneidabschnitt durch die auf ihn wirkenden Zerspanungskräfte, etwa entgegen der Federkraft einer Feder, insbesondere entgegen einem federvorgespannten Druckknopf, in der ersten (drehmomentgekoppelte) Axialposition gehalten. Wirken keine Zerspanungskräfte auf den Schneidabschnitt, wird der Schneidabschnitt, etwa durch die Federkraft der Feder, insbesondere durch den durch die Federvorspannung (nach vorne) gedrückten Druckknopf, in die zweite (drehmomententkoppelte) Axialposition verlagert. Da dem Schneidabschnitt keine Zerspanungskräfte entgegenwirken, sobald der Schädelknochen durchdrungen wurde, d.h., sobald der Trepanationsvorgang erfolgt ist, löst die Druckfeder sofort den Drehmomenteingriff zwischen dem Antriebsabschnitt und dem Schneidabschnitt. Der Schneidabschnitt ist demnach in einer drehmomententkoppelten Axialposition federvorgespannt.

Die US 2018/256 287 A1 offenbart eine Vorrichtung mit einem Handstück und einem damit verbindbaren Werkzeug, das ein elektronisches Bauteil in Form eines RFID-Transponders aufweist.

Die EP 2 581 061 A1 offenbart eine Vorrichtung mit einem Handstück und mit einem über eine Kupplungsvorrichtung damit koppelbaren Werkzeug, bei deren Kupplungseingriff ein Positionierungselement in eine Vertiefung zur drehfesten Verbindung formschlüssig eingreift, wodurch ein elektrischer Kontakt des Handstücks mit einem elektrischen Kontakt des Werkzeugs leitungsgebunden oder drahtlos verbunden wird.

Die WO 03/013372 A2 offenbart ein chirurgisches Werkzeug mit einem Handstück, das über eine interne Stromerzeugungseinheit verfügt, die zur Betätigung eines am Handstück angebrachten Zubehörteils verwendet wird. Im Inneren des Zubehörs befindet sich ein Identifikationschip, der die Betriebs- und/oder physikalischen Eigenschaften des Zubehörs beschreibt. Komplementäre Spulen im Handstück und im Zubehör erleichtern das Auslesen durch induktive Kopplung der Daten im Zubehör. Auf der Grundlage der gelesenen Daten betätigt die Steuerkonsole das Handstück in einer für das angebrachte Zubehör geeigneten Weise.

Die DE 29 16 221 B1 offenbart ein medizinisches Werkzeug gemäß der Präambel von Anspruch 1.

Das Werkzeug, insbesondere das Trepanationswerkzeug, kann als ein wiederverwendbares Werkzeug, d.h. als ein Werkzeug das für einen Mehrfachgebrauch mit Wiederaufbereitung geeignet ist, ausgebildet sein. Alternativ kann das (Trepanations-)Werkzeug als ein Einweg-Werkzeug (auch als Single-Use-Werkzeug bekannt) ausgebildet sein, d.h. als ein Werkzeug, das zum Einzelgebrauch/einmaligen Gebrauch geeignet ist.

Für den Anwender ist es oftmals nicht möglich, auf einfache Weise zu erkennen, um welches Werkzeug, beispielsweise welche Art von Werkzeugen, welche Größe des Werkzeugs und/oder welche Benutzungsart, d.h. Wiederverwendung oder Einweg-Werkzeug, es sich handelt. Auch ist es bisher nicht möglich, medizinische Werkzeuge, wie beispielsweise Handwerkzeuge oder Werkzeuge zum Einsetzen in chirurgischen (Hand-)Instrumenten, automatisch zu erkennen. Zur Identifizierung des Werkzeugs muss beispielsweise ein Label oder eine Umverpackung manuell gesichtet werden. Somit ist es nicht möglich, eine Fehlbenutzung des verwendeten Werkzeugs, etwa die Verwendung falscher Werkzeugparameter und/oder die Verwendung eines für die jeweilige medizinische Anwendung nicht geeigneten Werkzeugs, auszuschließen. Auch ist es aufgrund der fehlenden automatischen Dokumentation nicht möglich, eine verwendete Kombination von Werkzeugen oder eine Fehlbenutzung, etwa durch Überlastung des Werkzeugs, und damit verbundene Produktschäden nachzuvollziehen. Weiterhin kann der Werkzeugbestand ohne eine zeitaufwändige Inventur nicht erfasst werden. Ferner müssen die Werkzeuge vor ihrem Einsatz auf ihre Funktionsfähigkeit und ihren ordnungsgemäßen Zustand geprüft werden. Dazu muss die sichere Verbindung aller zu verwendenden Produkte, insbesondere der sichere Sitz des Werkzeugs im Handstück, geprüft werden, indem manuell am Werkzeug zur Überprüfung der Kupplung gezogen wird, was jedoch eine latente Verletzungsgefahr birgt.

Es ist also die Aufgabe der Erfindung, die Nachteile aus dem Stand der Technik zu vermeiden oder zu vermindern und ein medizinisches Werkzeug, insbesondere Trepanationswerkzeug, bereitzustellen, welches eine automatische Werkzeugerkennung und/oder automatische Werkzeugdokumentation ermöglicht und somit ein Risiko einer eventuellen Fehlbenutzung des Werkzeugs verringert.

### Zusammenfassung der Erfindung

Die Aufgabe der Erfindung wird durch den Gegenstand des unabhängigen Patentanspruchs gelöst. Vorteilhafte Weiterbildungen sind Gegenstand der Unteransprüche.

Genauer gesagt wird die Aufgabe der Erfindung durch ein medizinisches Werkzeug gelöst, das als ein drehantreibbares Werkzeug, insbesondere als ein spanabhebendes Werkzeug, vorzugsweise als ein Trepanationswerkzeug, ausgebildet ist und das einen mit einer medizinischen Vorrichtung zur insbesondere formschlüssigen Drehmomentübertragung verbindbaren Antriebsabschnitt (Drehmoment-Einleitabschnitt) und einen mit dem Antriebsabschnitt drehmomentübertragend koppelbaren Schneidabschnitt hat. Das Werkzeug weist eine (erste) elektronische Baugruppe auf, die ausgebildet ist, um durch ein Entkoppeln des Schneidabschnitts von dem Antriebsabschnitt aktiviert zu werden. Insbesondere ist der Schneidabschnitt relativ zu dem Antriebsabschnitt, etwa entgegen einem federvorgespannten Druckknopf, axial begrenzt zwischen einer ersten Axialposition, in der der Schneidabschnitt und der Antriebsabschnitt drehmomentgekoppelt sind, und einer zweiten Axialposition, in der der Schneidabschnitt von dem Antriebsabschnitt drehmomententkoppelt ist, verlagerbar. Dabei ist die (erste) elektronische Baugruppe so ausgebildet und angeordnet, dass sie in der ersten Axialposition deaktiviert ist und in der zweiten Axialposition aktiviert ist. Mit anderen Worten wird ein durch die begrenzte axiale Relativverlagerung definierter Schaltweg zwischen dem Antriebsabschnitt und dem Schneidabschnitt genutzt, um die elektronische Baugruppe zu aktivieren.

Dies hat den Vorteil, dass die Kupplungs- und/oder Entkopplungsvorgänge des (Trepanations-)Werkzeuges (automatisch) erfasst und/oder dokumentiert werden können. Vorteilhafterweise kann also die Anzahl der Entkopplungen je Operation mitgeschrieben werden. Dies hat auch den Vorteil, dass auch in nicht-stromversorgten Werkzeugen, bei denen Aufsätze von eigentlichen Abtrieb abgekoppelt werden, die Abkopplung zur Schließung oder Unterbrechung eines Stromkreises und damit zur Identifikation genutzt werden kann, was sich in vorteilhafter Weise auf die Digitalisierung von (medizinischen) Werkzeugen und Vorrichtung verwendet lässt.

Beispielsweise kann die (erste) elektronische Baugruppe durch das Entkoppeln des Schneidabschnitts von dem (Antriebsabschnitt) mechanisch so schaltbar sein, dass ein Stromkreis der elektronischen Baugruppe in einer betätigten Schaltstellung geschlossen (oder geöffnet) und in einer unbetätigten Schaltstellung geöffnet (oder geschlossen) wird.

Gemäß einer bevorzugten Ausführungsform kann die (erste) elektronische Baugruppe einen vorzugsweise als ein Taster ausgebildeten Schalter haben, der so von einem mit dem Schneidabschnitt drehkoppelbaren Betätigungsabschnitt mechanisch betätigbar ist, dass er die Anzahl der Umdrehungen des Schneidabschnitts, insbesondere nach dem Entkoppeln des Schneidabschnitts von dem Antriebsabschnitt, erfasst. Beispielsweise kann der vorzugsweise an einem nicht-drehendem Bauteil des Werkzeugs angeordnete Taster, etwa in Form einer Kuppe oder einer schiefen Ebene, so ausgebildet sein, dass er von einem mit dem Schneidabschnitt drehkoppelbaren Betätigungsabschnitt, etwa in Form einer Außenhülse, des Werkzeugs korrespondieren mit der Umdrehungszahl, beispielsweise bei jeder Umdrehung, des Schneidabschnitts mechanisch betätigt wird. Gemäß einer vorteilhaften Weiterbildung der bevorzugten Ausführungsform kann der Betätigungsabschnitt so ausgebildet sein, dass er in der ersten Axialposition von dem Schneidabschnitt drehentkoppelt und in der zweiten Axialposition mit dem Schneidabschnitt drehgekoppelt ist. Dadurch wird sichergestellt, dass sich der Betätigungsabschnitt nur (mit dem Schneidabschnitt) mitdreht, wenn der Schneidabschnitt von dem Antriebsabschnitt entkoppelt ist. So werden nur die Umdrehungen nach dem Entkoppeln erfasst. Erfahrungsgemäß hat sich die Anzahl der (Rest-)Umdrehungen nach dem Entkoppeln als besonders aussagekräftig bezüglich der Abnutzung und der Lebensdauer des Werkzeugs erwiesen. Vorzugsweise wird die Anzahl der Entkopplungen und/oder die Anzahl der Restumdrehungen in einer Speichereinrichtung der elektronischen Baugruppe gespeichert und/oder vorzugsweise in Kombination mit werkzeugspezifischen Daten, wie der Seriennummer oder Artikelnummer, an die externe Verarbeitungseinheit übermittelt. Beispielsweise kann die Informationsweitergabe in Echtzeit oder bedarfsgerecht (Just-In-Time) erfolgen. Mit anderen Worten ist der Betätigungsabschnitt so angeordnet und ausgebildet, dass der Schalter/Taster korrespondierend mit der Umdrehungszahl des Schneidabschnitts betätigt wird. Beispielsweise kann der Betätigungsabschnitt durch mehrere Rastelemente, insbesondere in Umfangsrichtung verteilt angeordnete Rastelemente, gebildet sein, so dass der Schalter/Taster je Umdrehung mehrfach betätigt. So können auch nicht vollständige Umdrehungen des Schneidabschnitts erfasst werden. Durch mehrere Rastelemente ist gleichzeitig eine mechanische Verrastung gewährleistet. Mit anderen Worten entspricht die Anzahl der Umdrehungen beispielsweise einem Quotienten aus der Anzahl der Betätigungen des zweiten Schalters und der Anzahl der Rastelemente.

Vorzugsweise kann die (erste) elektronische Baugruppe zur Erzeugung einer Funkverbindung eine in einem Kunststoffbauteil des Werkzeugs angeordnete Kommunikationsvorrichtung aufweisen oder mit der Kommunikationsvorrichtung einer zweiten elektronischen Baugruppe verbunden sein, die weiter bevorzugt so ausgebildet ist, dass sie bei Aktivierung der (ersten) elektronischen Baugruppe ein Funksignal mit Daten über den Entkupplungsvorgang überträgt. Vorzugsweise weist die (erste) elektronische Baugruppe eine (erste) Speichereinrichtung auf oder ist mit der Speichereinrichtung der zweiten elektronischen Baugruppe verbunden, um die Anzahl der Aktivierungen der (ersten) elektronischen Baugruppe (und somit die Anzahl der Entkupplungen des Schneidabschnitts) und/oder die Anzahl der (Rest-)Umdrehungen zu erfassen.

Gemäß einer bevorzugten Ausführungsform kann die (erste) elektronische Baugruppe in einem feststehenden Bauteil des Werkzeugs angeordnet sein. Dies hat den Vorteil, dass die elektronische Baugruppe bei der zerspanenden Bearbeitung nicht mitgedreht werden muss.

Gemäß einer bevorzugten Ausführungsform kann Werkzeug eine weitere elektronische Baugruppe aufweisen, die ausgebildet ist, um durch ein Verbinden des Werkzeugs mit der medizinischen Vorrichtung, vorzugsweise durch einen Steckvorgang des Werkzeugs in die medizinische Vorrichtung, aktiviert zu werden. Das Werkzeug als solches ist demnach mit einer elektronischen Baugruppe zum Erfassen von Informationen ausgestattet. Dabei kann die elektronische Baugruppe automatisch aktiviert werden, indem das Werkzeug in die medizinische Vorrichtung gesteckt wird.

Mit anderen Worten weist das Werkzeug (Trepanationswerkzeug) eine darin integrierte elektronische Baugruppe auf, die aktiviert/betätigt ist, wenn das Werkzeug mit der Vorrichtung (chirurgisches Handstück) verbunden/gekoppelt ist, insbesondere in die Vorrichtung eingesteckt ist, und die deaktiviert/unbetätigt ist, wenn das Werkzeug und die Vorrichtung unverbunden/voneinander entkoppelt sind, insbesondere das Werkzeug nicht in die Vorrichtung eingesteckt ist. Das heißt, dass eine Aktivierung der elektronischen Baugruppe von einer Kopplung des Werkzeugs und der Vorrichtung abhängig ist, wodurch vorteilhafterweise eine Werkzeugkopplung automatisch erkannt werden kann. Dies hat auch den Vorteil, dass der sichere Sitz zwischen dem Werkzeug und der Vorrichtung nicht mehr händisch, etwa durch Ziehen am Werkzeug, überprüft werden muss, da eine Rückmeldung über das erfolgreiche Koppeln durch die Aktivierung der elektronischen Baugruppe erfolgen kann.

Gemäß einer vorteilhaften Weiterbildung kann die weitere elektronische Baugruppe eine weitere Speichereinrichtung haben, in der werkzeugspezifische Daten, wie etwa Werkzeugbetriebsparameter, Werkzeugzustandsdaten, Anwendungsparameter, Seriennummer, Artikelnummer, Mindesthaltbarkeitsdatum (MHD), Chargennummer (LOT) und/oder weitere Informationen, gespeichert sind, die bei Aktivierung der weiteren elektronischen Baugruppe vorzugsweise an eine externe Verarbeitungseinheit und/oder an einen Anwender des Werkzeugs übermittelt und/oder ausgegeben werden. Das heißt, dass die werkzeugspezifischen Daten, insbesondere zur Erkennung des Werkzeugs, bei Aktivierung der weiteren elektronischen Baugruppe extern erfassbar bereitgestellt werden. Mit anderen Worten können durch die Aktivierung der weiteren elektronischen Baugruppe die gespeicherten Daten übermittelt und weiterverarbeitet werden, so dass eine automatische Werkzeugerkennung bereitgestellt werden kann. Die Verarbeitungseinheit kann beispielsweise ein Endgerät, wie ein Tablet oder ein Smartphone, oder ein Steuergerät oder eine internetbasierte Plattform, wie eine Cloud, sein. Durch die automatische Werkzeugerkennung kann gleichzeitig eine automatische Dokumentation realisiert werden. Zudem ist es dadurch möglich, basierend auf den übermittelten werkzeugspezifischen Daten individuelle Werkzeugparameter, wie Drehzahl oder Bestromung, automatisiert einzustellen, was eine Fehlbenutzung verhindert. Ferner kann beispielsweise eine Mehrfachverwendung des Werkzeugs erfasst werden. So kann dem Anwender etwa mitgeteilt werden, wenn das Werkzeug nur für den Einmal-Gebrauch geeignet ist, aber das Werkzeug bereit eingesetzt wurde. Diese Informationen werden gespeichert. Der Anwender kann ggf. auch intraoperativ informiert werden.

Gemäß einer bevorzugten Ausführungsform kann die weitere elektronische Baugruppe in einem feststehenden Bauteil des Werkzeugs angeordnet sein. Dies hat den Vorteil, dass die elektronische Baugruppe bei der zerspanenden Bearbeitung nicht mitgedreht werden muss.

Gemäß einer bevorzugten Ausführungsform kann die weitere elektronische Baugruppe einen weiteren Schalter haben, der durch das Verbinden des Werkzeugs mechanisch betätigbar ist. Mit anderen Worten ist der Schalter an dem mit der Vorrichtung zu verbindenden Antriebsabschnitt des Werkzeugs so ausgebildet, dass der Schalter in einem unbetätigten Zustand von dem Antriebsabschnitt (axial oder radial) hervorsteht und durch das Verbinden des Antriebsabschnitts mit der medizinischen Vorrichtung relativ zu dem Antriebsabschnitt verlagert wird, insbesondere in den Antriebsabschnitt zur Betätigung des Schalters hineingedrückt wird. Vorzugsweise weist der Antriebsabschnitt eine standardisierte Schnittstelle, wie etwa einen Hudson-Anschluss, auf. Durch die Anordnung des mechanisch zu betätigenden Schaltern an dem Antriebsabschnitt wird der Schalter automatisch betätigt, wenn das Werkzeug in die medizinische Vorrichtung eingesteckt wird, da das Werkzeug zur Gewährleistung eines sicheren Sitzes passgenau von der Vorrichtung aufgenommen werden muss. Daher wird der Schalter bei Verwendung mit jeder (herkömmlichen) Vorrichtung mechanisch betätigt, sofern der Antriebsabschnitt im Bereich des Schalters an der Vorrichtung anliegt. Da die Schnittstelle zwischen dem Werkzeug und der Vorrichtung üblicherweise standardisiert ist, erfolgt die Aktivierung der elektronischen Baugruppe unabhängig von dem sonstigen Aufbau der Vorrichtung.

Gemäß einer vorteilhaften Weiterbildung der bevorzugten Ausführungsform kann der Schalter durch das Verbinden des Werkzeugs mechanisch so betätigbar sein, dass der Schalter einen Stromkreis der elektronischen Baugruppe in einer betätigten Schaltstellung schließt und in einer unbetätigten Schaltstellung öffnet. Vorzugsweise weist die elektronische Baugruppe eine Kommunikationsvorrichtung zur Erzeugung einer Funkverbindung auf, die bei Aktivierung der elektronischen Baugruppe, also bei Schließen des Stromkreises, ein Funksignal mit den werkzeugspezifischen Daten überträgt. Beispielsweise kann die Kommunikationsvorrichtung das Funksignal aktiv, etwa durch WLAN oder Bluetooth Low Energy (BLE) oder durch ein Low-Power-Wireless-Netzprotokoll, wie LoRaWAN (Long Range Wide Area Network), oder passiv, etwa durch RFID oder NFC, übertragen. Das Funksignal kann auch durch einen anderen Funkstandard, der zur (kontaktlosen) Datenübertragung geeignet ist, übertragen werden und ist auf keine der genannten Funkstandards oder keine bestimmten Frequenzbereich beschränkt. Wenn der Stromkreis bei Entkopplung des Werkzeugs von der Vorrichtung unterbrochen wird, sendet die Kommunikationsvorrichtung kein Funksignal mehr, oder ist nicht mehr erreichbar im Falle von passiven Technologien. Mit anderen Worten kann die elektronische Baugruppe beispielweise einen RFID-Chip, einen NFC-Chip, ein WLAN-Modul und/oder einen Bluetooth Low Energy-Chip aufweisen, die jeweils ausgebildet sind, um das Funksignal beim Aktiveren der elektronischen Baugruppe an einen zugeordneten in einer Umgebung des Werkzeugs befindlichen Empfänger zu übertragen. Von dem Empfänger können die durch das Funksignal übertragenen Daten an weitere Endgeräte weitergeleitet werden. Gemäß einer weiter bevorzugten Weiterbildung der bevorzugten Ausführungsform kann die Kommunikationsvorrichtung in einem Kunststoffgehäuse des Werkzeugs angeordnet sein, wodurch in vorteilhafter Weise eine Funkdurchlässigkeit gegeben ist.

Gemäß einer bevorzugten Ausführungsform kann der Schalter durch das Verbinden des Werkzeugs zwischen der betätigten Schaltstellung und der unbetätigten Schaltstellung in Axialrichtung verlagerbar sein. Eine Einsteckrichtung des Werkzeugs entspricht üblicherweise der Axialrichtung, so dass eine axiale Betätigung des Schalters auf einfache Weise realisiert werden kann. Der Schalter kann vorzugsweise von einer axialen Anschlagsfläche des Werkzeugs hervorstehen, an der die Vorrichtung im gekoppelten Zustand anliegt. So kann sichergestellt werden, dass der Schalter nur in einer Endposition des Werkzeugs in der Vorrichtung, in der das Werkzeug und die Vorrichtung axialfest verbunden sind, betätigt wird, um zu vermeiden, dass die elektronische Baugruppe aktiviert wird, wenn der Steckvorgang noch nicht abgeschlossen ist und das Werkzeug keinen sicheren Sitz hat.

Gemäß einer alternativen bevorzugten Ausführungsform kann der Schalter durch das Verbinden des Werkzeugs zwischen der betätigten Schaltstellung und der unbetätigten Schaltstellung in Radialrichtung verlagerbar sein. Der Schalter kann vorzugsweise von einer radialen Außenumfangsfläche des Antriebsabschnitts hervorstehen, an der die Vorrichtung im gekoppelten Zustand anliegt. Da der Antriebsabschnitt oftmals so in die Vorrichtung eingesteckt wird, dass er radial außen an dem radialen Innendurchmesser der Vorrichtung anliegt, kann bei dem radial zu betätigenden Schalter eine automatische Betätigung durch das Verbinden des Werkzeugs mit der Vorrichtung sichergestellt werden.

Gemäß einer bevorzugten Ausführungsform kann die elektronische Baugruppe eine Rückmeldevorrichtung aufweisen und/oder mit einer externen Rückmeldeeinrichtung verbindbar sein. Die Rückmeldevorrichtung und/oder die Rückmeldeeinrichtung können/kann insbesondere so ausgebildet sein, dass eine akustische und/oder visuelle Rückmeldung ausgegeben wird, wenn die elektronische Baugruppe aktiviert wird oder aktiviert ist. So erhält der Anwender eine automatische Bestätigung über das erfolgreiche Verbinden, so dass der sichere Sitz nicht mehr durch Berühren überprüft werden muss. Beispielsweise kann die Rückmeldevorrichtung als ein Leuchtmittel, wie eine vorzugsweise in dem Antriebsabschnitt von außen sichtbare LED, und/oder als ein akustischer Signalgeber ausgebildet sein, deren Leuchten und/oder Ton Rückmeldung darüber gibt, ob das Werkzeug mit der Vorrichtung (korrekt) verbunden ist oder nicht, d.h. ein erfolgreiches Verbinden bestätigt. Beispielsweise kann die externe Rückmeldeeinrichtung als ein insbesondere über die Funkverbindung gekoppeltes Steuergerät oder Endgerät, wie ein Smartphone oder ein Tablet, ausgebildet sein, das eine Meldung darüber gibt, ob das Werkzeug mit der Vorrichtung (korrekt) verbunden ist oder nicht.

Gemäß einem weiteren Aspekt der Erfindung kann der Antriebsabschnitt einen vorzugsweise aus Kunststoff ausgebildeten Sockel, der insbesondere direkt mit der medizinischen Vorrichtung verbindbar ist, und einen vorzugsweise aus Metall ausgebildeten Mitnehmer haben, der axialgesichert und formschlüssig drehfest mit dem Sockel verbunden ist. Insbesondere kann die (erste) elektronische Baugruppe in dem Sockel angeordnet sein. Alternativ können der Sockel und der Mitnehmer aus Kunststoff ausgebildet sein. Weiter alternativ kann der Sockel aus Metall und der Mitnehmer aus Kunststoff ausgebildet sein.

Gemäß einer bevorzugten Ausführungsform kann der Sockel einen Axialsicherungsabschnitt, etwa in Form von Rastbuchten, besitzen, in welchen ein an dem Mitnehmer ausgebildeter Gegensicherungsabschnitt, etwa in Form von Rasthaken, zur Axialsicherung eingreift. Gemäß einer bevorzugten Ausführungsform kann der Sockel einen Drehmomentübertragungsabschnitt, etwa in Form von Kraftübertragungsbuchten, besitzen, in welchen ein an dem Mitnehmer ausgebildeter Gegenabschnitt, etwa in Form von Stegen, zur formschlüssigen Drehmomentübertragung eingreift. Mit anderen Worten sind die Funktionen der Axialsicherung und der Drehmomentübertragung an separaten Abschnitten des Mitnehmers ausgebildet. Vorzugsweise sind die Rastbuchten und/oder die Kraftübertragungsbuchten symmetrisch angeordnet. Gemäß einer besonders bevorzugten Ausführungsform kann der Axialsicherungsabschnitt distal zum Drehmomentübertragungsabschnitt angeordnet sein, um einen geeigneten Kraftfluss zu ermöglichen. Der Drehmomentübertragungsabschnitt ist also näher zu dem Schneidabschnitt als der Axialsicherungsabschnitt angeordnet.

Gemäß einem weiteren Aspekt der Erfindung kann der Schneidabschnitt einen vorzugsweise mit dem Antriebsabschnitt direkt gekoppelten Basisabschnitt und einen separat davon ausgebildeten, vorzugsweise die Schneiden tragenden Eingriffsabschnitt aufweisen, der an einer (ersten) Schnittstelle, etwa in Form eines Gewindes, mit dem Basisabschnitt axialfest und/oder drehfest verbunden ist. Vorzugsweise ist die Schnittstelle so ausgebildet, dass der Eingriffsabschnitt durch Drehen entgegen seiner Drehrichtung im Zerspanungseingriff mit dem Basisabschnitt angebracht wird, um ein unbeabsichtigtes Lösen durch die Zerspanungskräfte zu vermeiden. Besonders bevorzugt ist die Schnittstelle so ausgebildet, dass sie mit Eingriffsabschnitten unterschiedlicher Bauart und/oder Größe verbindbar ist. Weiter bevorzugt kann der Schneidabschnitt einen separat von dem Eingriffsabschnitt ausgebildeten Hülsenabschnitt aufweisen, der an einer (zweiten) Schnittstelle an einem Außendurchmesser des Eingriffsabschnitts angebracht ist. Insbesondere können die Eingriffsabschnitte unterschiedlicher Bauart und/oder Größe denselben Außendurchmesser besitzen. Durch den modularen Aufbau und die Verwendung von Gleichteilen bei unterschiedlichen Werkzeugarten und/oder Werkzeuggrößen können die Herstellungskosten des Werkzeugs reduziert werden.

Gemäß einem weiteren Aspekt der Erfindung kann der Schneidabschnitt einen Kunststoffdom aufweisen, an dem die Schneiden bildende Einlegeteile, insbesondere aus Metall, etwa durch Wärmeprägen, fest angebracht sind. Dadurch kann abgesehen von den durch Metallblättern gebildeten Schneiden das gesamte (Trepanations-) Werkzeug aus Kunststoff kostengünstig hergestellt werden.

### Kurzbeschreibung der Figuren

Fign 1a und 1b sind perspektivische Darstellungen eines Verbindens eines erfindungsgemäßen Werkzeugs mit einer medizinischen Vorrichtung gemäß einer ersten Ausführungsform der Erfindung zur Aktivierung einer elektronischen Baugruppe.
Fign. 2a und 2b zeigen perspektivische Darstellungen des Werkzeugs mit einem Schalter der elektronischen Baugruppe in zwei unterschiedlichen Ausführungsformen.
Fign. 3 und 4 zeigen schematische Darstellungen einer Kommunikationsvorrichtung der elektronischen Baugruppe des Werkzeugs.
Fign. 5 und 6 zeigen perspektivische Darstellungen des Werkzeugs in der ersten Ausführungsform.
Fign. 7 bis 10 zeigen perspektivische Darstellungen eines Antriebsabschnitts des Werkzeugs und seiner Einzelteile.
Fign. 11 bis 13 sind verschiedene perspektivische, teilweise geschnittene Darstellungen des Werkzeugs in einer zweiten Ausführungsform.
Fign. 14 bis 16 sind perspektivische Darstellungen eines Schneidabschnitts des Werkzeugs in verschiedenen Größen.
Fign. 17 und 18 sind perspektivische Darstellungen des Werkzeugs in einer weiteren Ausführungsform.

Nachstehend werden Ausführungsbeispiele der vorliegenden Offenbarung auf der Basis der zugehörigen Figuren beschrieben. Die Figuren sind lediglich schematischer Natur und dienen dem Verständnis der Erfindung. Gleiche Elemente sind mit denselben Bezugszeichen gekennzeichnet.

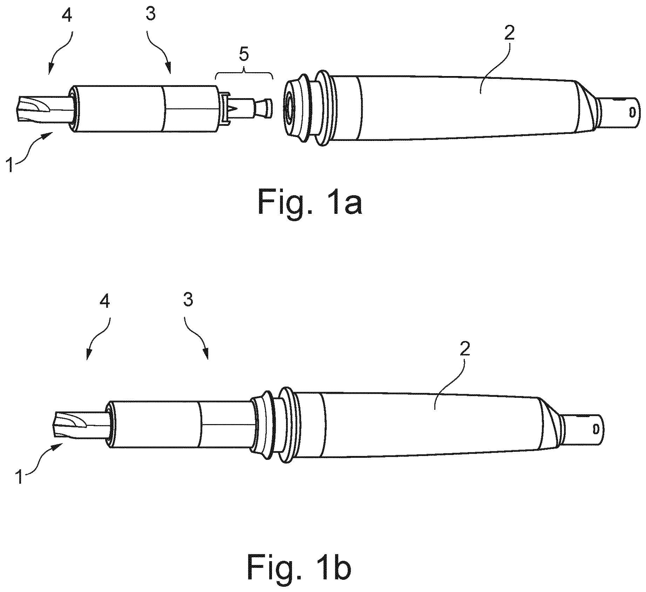

Fig. 1 zeigt ein medizinisches Werkzeug 1, das mit einer medizinischen Vorrichtung 2, wie einem chirurgischen Handstück, verbindbar ist. In Fig. 1a ist das Werkzeug 1 nicht mit der medizinischen Vorrichtung 2 verbunden, d.h. von der Vorrichtung 2 abgekoppelt. In Fig. 1b ist das Werkzeug 1 mit der medizinischen Vorrichtung 2 verbunden, d.h. mit der Vorrichtung 2 gekoppelt. In den dargestellten Ausführungsformen ist das Werkzeug 1 als ein drehantreibbares Werkzeug ausgebildet. Dazu ist das Werkzeug 1 zur Übertragung eines Drehmoments zum Antrieb des Werkzeugs 1 mit der Vorrichtung 2 verbunden. Insbesondere kann das Werkzeug 1 als ein spanabhebendes Werkzeug ausgebildet sein.

Das Werkzeug 1 weist einen Antriebsabschnitt 3 auf, der teilweise in die Vorrichtung 2 eingesteckt ist und in die Vorrichtung 2 formschlüssig drehgesichert eingreift. Ferner weist das Werkzeug 1 einen den Antriebsabschnitt 3 in Axialrichtung verlängernden Schneidabschnitt 4 auf. An dem Schneidabschnitt 4 sind Schneiden zur spanabhebenden Bearbeitung angeordnet. Der Antriebsabschnitt 3 weist einen Kupplungsabschnitt 5 auf, der den Teil des Antriebsabschnitts 3 bildet, der im verbundenen Zustand vollständig in die Vorrichtung 2 eingesteckt ist. Der Kupplungsabschnitt 5 ist in den dargestellten Ausführungsformen als ein Hudson-Anschluss 6 ausgebildet, der universell als Schnittstelle für Handstücke verwendet wird.

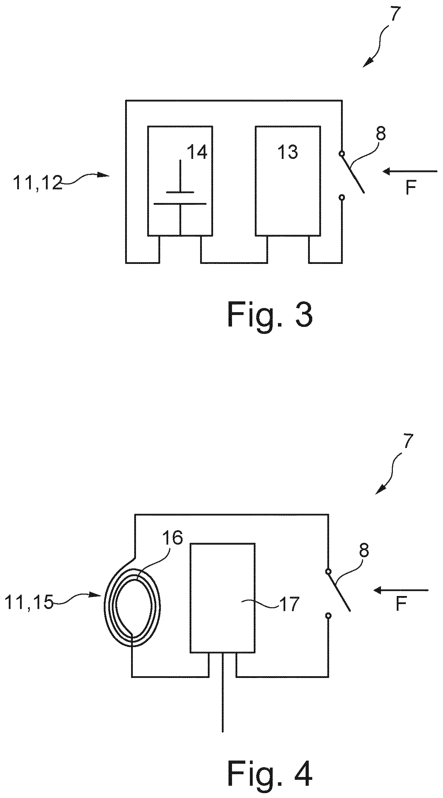

Das Werkzeug 1 weist eine elektronische Baugruppe 7 (vgl. Fign. 3 und 4) auf. Die elektronische Baugruppe 7 ist ausgebildet, um durch ein Verbinden des Werkzeugs 1 mit der medizinischen Vorrichtung 2, vorzugsweise durch einen Steckvorgang des Werkzeugs 1 in die medizinische Vorrichtung 2, aktiviert zu werden. Vorzugsweise hat die elektronische Baugruppe 7 einen Schalter 8, der durch das Verbinden des Werkzeugs 1 mechanisch so betätigbar ist, dass der Schalter 8 einen Stromkreis der elektronischen Baugruppe 7 in einer ersten Schaltstellung schließt und in einer zweiten Schaltstellung öffnet.

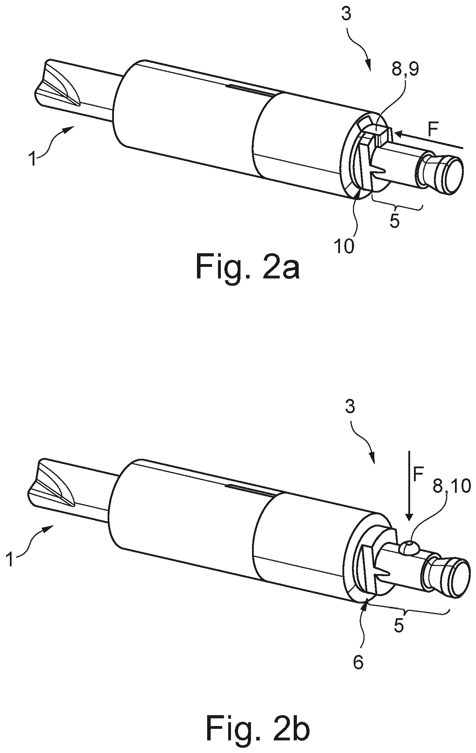

In Fig. 2a ist der Schalter 8 als ein Axialschalter 9 ausgebildet, der zur Betätigung in Axialrichtung verlagerbar ist. Der Axialschalter 9 ist an einer axialen Anlagefläche des Kupplungsabschnitts 5 angeordnet und steht axial in Richtung zu der Vorrichtung 2 hervor. An der Anlagefläche liegt die Vorrichtung 2 im mit dem Werkzeug 1 verbundenen Zustand an und betätigt somit den Axialschalter 9. Wenn das Werkzeug 1 mit der Vorrichtung 2 verbunden ist, ist der Axialschalter 9 betätigt. Wenn das Werkzeug 1 nicht mit der Vorrichtung 2 verbunden ist, ist der Axialschalter 9 unbetätigt. In Fig. 2b ist der Schalter 8 als ein Radialschalter 10 ausgebildet, der zur Betätigung in Radialrichtung verlagerbar ist. Der Radialschalter 10 weist eine halbkugelförmige, kuppelartige Form auf. Der Radialschalter 10 ist auf einem radialen Außenumfang des Kupplungsabschnitts 5 angeordnet und steht radial nach außen ab. Auf den radialen Außenumfang ist die Vorrichtung 2 im mit dem Werkzeug 1 verbundenen Zustand aufgeschoben und betätigt somit den Radialschalter 10. Wenn das Werkzeug 1 mit der Vorrichtung 2 verbunden ist, ist der Radialschalter 10 betätigt. Wenn das Werkzeug 1 nicht mit der Vorrichtung 2 verbunden ist, ist der Radialschalter 10 unbetätigt. Die Schalter 8 ist also so an dem Kupplungsabschnitt 5 angeordnet, dass er mechanisch durch die Vorrichtung 2 im verbundenen Zustand automatisch betätigt ist und im unverbundenen Zustand automatisch unbetätigt ist.

Die elektronische Baugruppe 7 kann eine Speichereinrichtung haben, in der werkzeugspezifische Daten, wie etwa Werkzeugbetriebsparameter, Werkzeugzustandsdaten, Anwendungsparameter, Seriennummer, Artikelnummer, Mindesthaltbarkeitsdatum (MHD), Chargennummer (LOT) und/oder weitere Informationen, gespeichert sind. Die elektronische Baugruppe 7 kann eine Kommunikationsvorrichtung 11 zur Erzeugung einer Funkverbindung haben. Die Kommunikationsvorrichtung 11 ist so angeordnet, dass sie bei Aktivierung der elektronischen Baugruppe 7, also bei Schließen des Stromkreises, ein Funksignal mit den in der Speichereinrichtung gespeicherten werkzeugspezifischen Daten überträgt.

Fig. 3 zeigt einen möglichen Aufbau der Kommunikationsvorrichtung 11, die als eine Bluetooth-Low-Energy-Einheit 12 ausgebildet ist. Alternativ kann die Kommunikationsvorrichtung 11 auch als ein anderes Funkmodul, etwa als ein W-LAN-Modul oder ein Lo-RA-WAN-Modul (Long Range Wide Area Network-Modul) ausgebildet sein. In der Kommunikationsvorrichtung 11 wird, wenn der Schalter 8 betätigt ist, der Stromkreis geschlossen und ein Bluetooth-Low-Energy-Chip 13 mit einer Batterie 14 verbunden. Die Bluetooth-Low-Energy-Einheit 12 kann ein Funksignal aktiv an einen zugeordneten in einer Umgebung des Werkzeugs 1 befindlichen Empfänger übertragen, wenn der Stromkreis geschlossen ist. Wenn der Stromkreis bei Entkopplung des Werkzeugs 1 von der Vorrichtung 2 unterbrochen wird, sendet die Kommunikationsvorrichtung 11 kein Funksignal mehr. Fig. 4 zeigt einen alternativen möglichen Aufbau der Kommunikationsvorrichtung 11, die als eine RFID- oder NFC-Einheit 15 ausgebildet ist. In der Kommunikationsvorrichtung 11 wird, wenn der Schalter 8 betätigt ist, der Stromkreis geschlossen und eine Spule 16 mit einem Speicher 17, zum Beispiel einem EEPROM (englisch für electrically erasable programmable readonly memory) verbunden. Im Gegensatz zu dem in Fig. 3 dargestellten Aufbau ist keine Batterie notwendig, wodurch die Lebensdauer der elektronischen Baugruppe 7 nicht von der Batterielebensdauer abhängt. Die RFID- oder NFC-Einheit 15 kann ein Funksignal passiv übertragen. Über die Spule 16 lässt sich der Speicher 17 auslesen. Das Funksignal kann durch die NFC-Einheit 15 beispielsweise an einen in der Vorrichtung 2 angeordneten Empfänger und von dort aus weitergegeben werden. Durch das Verbinden des Werkzeugs 1 mit der Vorrichtung können, wie in Fign. 3 und 4 schematisch gezeigt, in der Speichereinrichtung gespeicherte werkzeugspezifische Daten an Peripheriegeräte weitergegeben werden. Dort wiederum werden die Daten weiterverarbeitet und dem Anwender ausgegeben, in die Cloud gespeichert und/oder online gestellt.

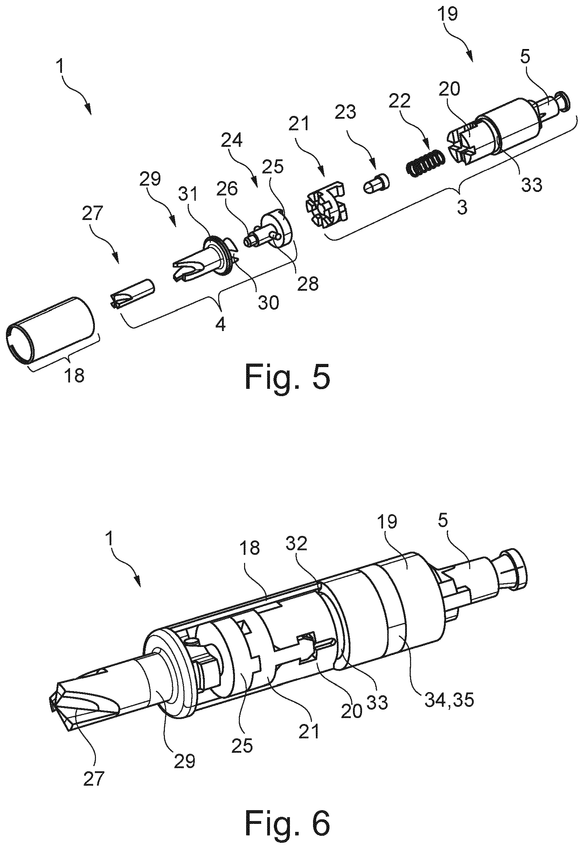

Ein Aufbau des Werkzeugs 1 wird mit Bezugnahme auf Fign. 5 und 6 erläutert. Das Werkzeug 1 kann funktional in den Antriebsabschnitt 3, den Schneidabschnitt 4 und einen Hülsenabschnitt 18 unterteilt werden.

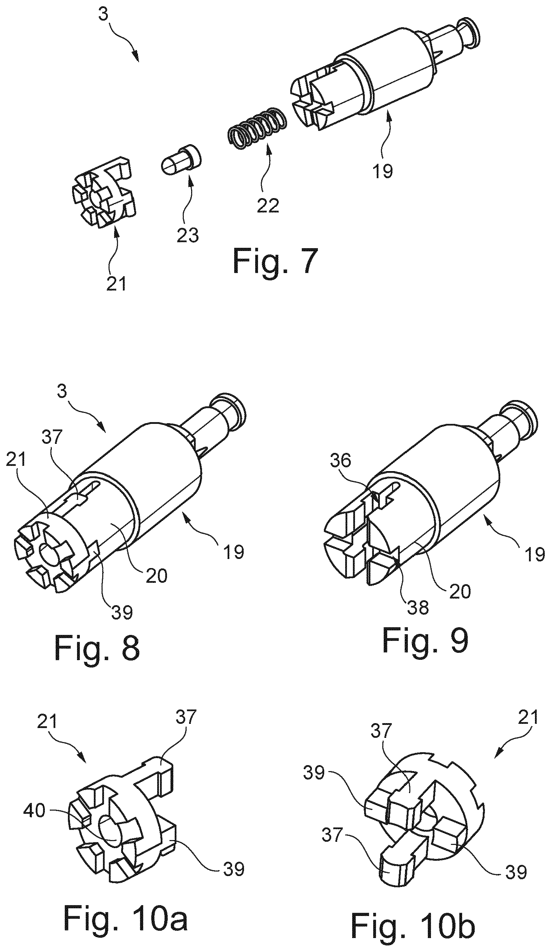

Der Antriebsabschnitt 3 (vgl. auch Fign. 7 bis 10) weist einen Sockel 19 auf, an dem der Kupplungsabschnitt 5 ausgebildet ist. Der Sockel 19 ist als ein Kunststoffbauteil ausgebildet. In dem Sockel 19 ist die elektronische Baugruppe 7 untergebracht. An einem proximalen Ende des Sockels 19 ist ein Verbindungsabschnitt 20 ausgebildet. Der Antriebsabschnitt 3 weist einen Mitnehmer 21 auf, der mit dem Verbindungsabschnitt 20 axialgesichert und drehfest verbunden ist. In dem Verbindungsabschnitt 20 ist eine Feder 22 aufgenommen, entgegen deren Federkraft der Schneidabschnitt 4 axial zwischen der erste Axialposition und der zweiten Axialposition verlagerbar ist. Axial zwischen dem Mitnehmer 21 und dem Verbindungsabschnitt 20 ist ein Druckknopf 23 angeordnet. Der Druckknopf 23 durchgreift axial eine zentrale Aussparung in dem Mitnehmer 21.

Der Schneidabschnitt 4 weist einen Basisabschnitt 24 auf. Der Basisabschnitt 24 dient zur Kraftübertragung/Drehmomentübertragung und weist einen Mitnehmer 25 auf. Der Mitnehmer 25 des Schneidabschnitts 4 kann in den Mitnehmer 21 des Antriebsabschnitts 3 formschlüssig drehgesichert eingreifen, so dass ein Drehmoment von dem Antriebsabschnitt 3 auf den Schneidabschnitt 4 übertragen werden kann. In der ersten Axialposition greift der Mitnehmer 25 in den Mitnehmer 21 ein. Der Basisabschnitt 24 weist eine erste Schnittstelle 26 auf. Über die erste Schnittstelle 26 kann ein erster Eingriffsabschnitt 27 des Schneidabschnitts 4, hier in Form eines Innenfräsers, mit dem Basisabschnitt 24 drehmomentübertragend verbunden werden. Die erste Schnittstelle 26 ist als ein Gewinde ausgebildet, auf das der erste Eingriffsabschnitt 27 vorzugsweise entgegen der Zerspanungsrichtung/Antriebsrichtung des Werkzeugs 1 aufgeschraubt werden kann. Insbesondere ist der erste Eingriffsabschnitt 27 durch ein Anzugsmoment gesichert. Der Basisabschnitt 24 weist eine zweite Schnittstelle 28 auf. Über die zweite Schnittstelle 28 kann ein zweiter Eingriffsabschnitt 29 des Schneidabschnitts 4, hier in Form eines Außenfräsers, mit dem Basisabschnitt 24 drehmomentübertragend verbunden werden. Die zweite Schnittstelle 28 ist als ein Querstift ausgebildet, auf den der zweite Eingriffsabschnitt 29 aufgeschoben werden kann. In dem zweiten Eingriffsabschnitt 29 ist eine Nut 30 ausgebildet, die den zweiten Eingriffsabschnitt 29 formschlüssig drehfest mit dem Querstift verbindet. Der zweite Eingriffsabschnitt 29 weist einen radial nach außen abstehenden Flansch 31 auf. Der Flansch 31 ist radial umlaufend ausgebildet.

Der Hülsenabschnitt 18 ist vorzugsweise aus Kunststoff aufgebaut. Der Hülsenabschnitt 18 bildet einen Außendurchmesser des Werkzeugs 1. Der Hülsenabschnitt 18 liegt an seinem proximalen Ende an dem Flansch 31 des Schneidabschnitts 4 an. Der Hülsenabschnitt 18 liegt an seinem distalen Ende an einer durch den Sockel 19 gestellten axialen Anschlagsfläche an. Der Hülsenabschnitt 18 weist einen radial nach innen abstehenden Zapfen 32 auf, der in eine umlaufende Nut 33 in dem Verbindungsabschnitt 20 eingreift und den Hülsenabschnitt 18 dadurch axial sichert.

In der in Fig. 6 dargestellten Ausführungsform weist die elektronische Baugruppe 7 eine Rückmeldevorrichtung 34 auf. Die Rückmeldevorrichtung 34, hier in Form einer LED 35, ist in dem Sockel 19 angeordnet und leuchtet, wenn die elektronische Baugruppe 7 aktiviert ist. Beispielsweise kann die LED 35 ein grünes Licht ausgeben, wenn sie korrekt gekuppelt ist, und ein rotes Licht ausgeben, wenn sie unvollständig gekuppelt ist. Die LED 35 kann auch ein blinkendes Licht oder ein durchgehend leuchtendes Licht ausgeben. Die LED 35 kann auch in anderen Farben leuchten. Die Rückmeldevorrichtung 34 kann beispielsweise auch mehrere LEDs aufweisen, von denen eine Rückmeldung über das erfolgreiche Kuppeln gibt und eine Rückmeldung über fehlerhaftes Kuppeln gibt. Alternativ oder zusätzlich kann die Rückmeldevorrichtung 34 eine akustische Rückmeldung ausgeben, wenn die elektronische Baugruppe 7 aktiviert wird oder aktiviert ist. Beispielsweise kann die Frequenz/Tonhöhe der akustische Rückmeldung oder der Zeitabstand zwischen mehreren akustischen Signalen/Rückmeldungen bei erfolgreichem Kuppeln unterschiedlich sein als bei nicht erfolgreichem Kuppeln.

Ein Aufbau des Antriebsabschnitts 3 wird mit Bezugnahme auf Fign. 7 bis 10 beschrieben. Der Antriebsabschnitt 3 wird insbesondere durch den als Metallbauteil ausgebildeten Mitnehmer 21 und den als Kunststoffbauteil ausgebildeten Sockel 19 gebildet. Alternativ könnten der Sockel 19 und der Mitnehmer 21 als Metallbauteil ausgebildet sein. Weiter alternativ könnten der Sockel 19 und der Mitnehmer 21 als Kunststoffbauteil ausgebildet sein. Ferner alternativ könnte der Sockel als Metallbauteil und der Mitnehmer 21 als Kunststoffbauteil ausgebildet sein. Die Feder 22 und der Druckknopf 23 sind für die Übertragung der Kräfte und Drehmomente unerheblich.

Der Sockel 19 und der Mitnehmer 21 sind axialgesichert miteinander verbunden. Dazu weist der Sockel 19 eine bzw. mehrere Rastbuchten 36 auf, in die ein bzw. mehrere Rasthaken 37 des Mitnehmers 21 eingreifen. Der Mitnehmer 21 hintergreift demnach in Axialrichtung den Sockel 19. Die Rastbuchten 36 sind symmetrisch, d.h. in Umfangsrichtung gegenüberliegend angeordnet. Die Rasthaken 37 sind symmetrisch, d.h. in Umfangsrichtung gegenüberliegend angeordnet. Der Sockel 19 und der Mitnehmer 21 sind drehmomentübertragend miteinander verbunden. Dazu weist der Sockel 19 eine bzw. mehrere Kraftübertragungsbuchten 38 auf, in die ein bzw. mehrere Stege 39 des Mitnehmers 21 eingreifen. Die Kraftübertragungsbuchten 38 sind symmetrisch, d.h. in Umfangsrichtung gegenüberliegend angeordnet. Die Stege 39 sind symmetrisch, d.h. in Umfangsrichtung gegenüberliegend angeordnet. Die Stege 39 liegen in Umfangsrichtung zwischen den Rasthaken 38. Der Mitnehmer 21 weist eine zentrale Aussparung 40 auf, durch die drei Druckknopf 23 zur Entkopplung, d.h. zum Außer-Eingriff-Bringen des Schneidabschnitts 4, durchgreifen kann.

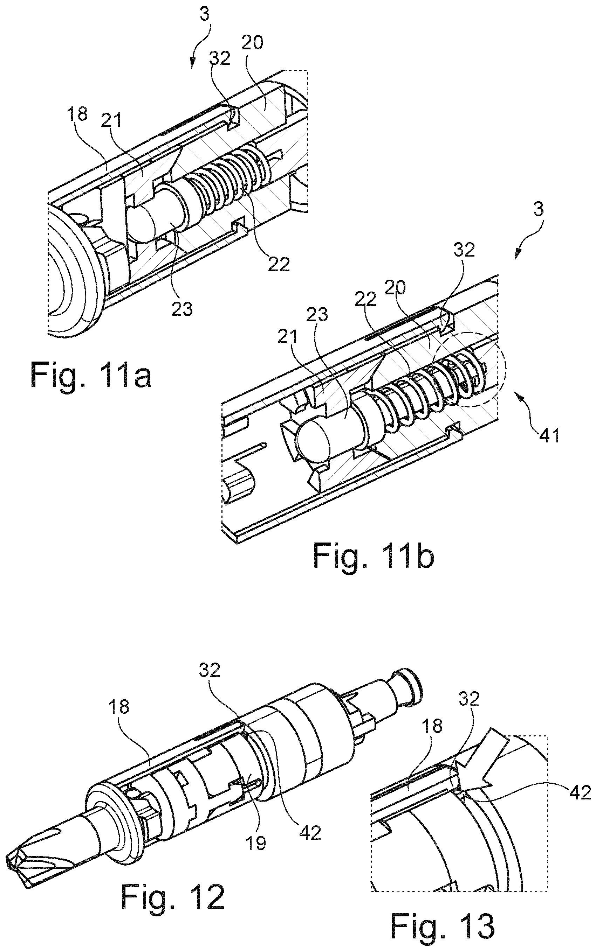

Das Werkzeug 1 ist in den dargestellten Ausführungsformen als ein Trepanationswerkzeug ausgebildet. Die Funktionsweise eines Trepanationswerkzeugs wird mit Bezugnahme auf Fign. 11a und 11b erläutert. Bei dem Werkzeug 1 ist der Schneidabschnitt 4 relativ zu dem Antriebsabschnitt 3 axial begrenzt zwischen einer ersten Axialposition (vgl. Fig. 11a), in der der Schneidabschnitt 4 und der Antriebsabschnitt 3 drehmomentgekoppelt sind, und einer zweiten Axialposition (vgl. Fig. 11b), in der der Schneidabschnitt 4 von dem Antriebsabschnitt 3 drehmomententkoppelt ist, verlagerbar. Dadurch kann der Schneidabschnitt 4 von dem eigentlichen Antrieb abgekoppelt werden. Im Zerspanungseingriff wird der Schneidabschnitt 4 durch die auf ihn wirkenden Zerspanungskräfte entgegen der Federkraft der Feder 22 in die erste Axialposition gedrückt. Der in dem Antriebsabschnitt 3 gelagerte Druckknopf 23 wird axial verlagert und die Feder 22 vorgespannt. Wirken keine Zerspanungskräfte auf den Schneidabschnitt 4, wird der Schneidabschnitt 4 durch die Federkraft der Feder 22 in die zweite Axialposition gedrückt. Durch die Federvorspannung wird der Druckknopf 23 in Richtung zu dem Schneidabschnitt 4 verlagert, so dass er den (abtriebsseitigen/drehmomentaufnehmenden) Mitnehmer 25 des Schneidabschnitts 4 außer Eingriff mit dem (antriebsseitigen/drehmomentweitergebenden) Mitnehmer 21 des Antriebsabschnitts 3 drückt.

In Fig. 11b ist durch einen gestrichelten Kreis die Axialrelativbewegung eines Schaltwegs zwischen der ersten Axialposition und der zweiten Axialposition gekennzeichnet. Diese Axialrelativbewegung kann genutzt werden, um eine zweite elektronische Baugruppe 41 zu aktivieren. Gemäß der Erfindung weist das Werkzeug 1 die zweite elektronische Baugruppe 41 auf, die ausgebildet ist, um durch ein Entkoppeln des Schneidabschnitts 4 von dem Antriebsabschnitt 3 aktiviert zu werden. Das heißt, dass die zweite elektronische Baugruppe 41 so ausgebildet ist, dass sie deaktiviert ist, wenn der Schneidabschnitt 4 relativ zu dem Antriebsabschnitt 3 in der (gekoppelten) Axialposition ist, und aktiviert ist, wenn der Schneidabschnitt 4 relativ zu dem Antriebsabschnitt 3 in der zweiten (entkoppelten) Axialposition ist. Insbesondere weist die zweite elektronische Baugruppe 4 einen nicht dargestellten Schalter auf, der durch die Axialverlagerung des Schneidabschnitts 4, insbesondere des Druckknopfs 23, mechanisch betätigt wird.

In Fign. 12 und 13 ist eine weitere Ausführungsform des Werkzeugs 1 dargestellt. Die zweite elektronische Baugruppe 41 hat einen zweiten Schalter 42. Der zweite Schalter 42 ist als ein Taster ausgebildet. Der zweite Schalter 42 ist so von dem Schneidabschnitt 4 mechanisch betätigbar, dass er die Anzahl der Umdrehungen des Schneidabschnitts 4, insbesondere nach dem Entkoppeln des Schneidabschnitts 4 von dem Antriebsabschnitt 3, erfasst. Der zweite Schalter 42 weist eine halbkugelförmige, kuppelartige Form auf. Der zweite Schalter 42 kann auch in Form einer schiefen Ebene gebildet sein. Der zweite Schalter 42 ist auf einem radialen Außenumfang des Antriebsabschnitts 3, hier in einem Bereich des Verbindungsabschnitts 20, angeordnet und steht radial nach außen ab. Der zweite Schalter 42 ist in der Nut 33 angeordnet. Der Zapfen 32 an dem Hülsenabschnitt 18 dient als ein Betätigungsabschnitt, so dass der zweite Schalter 42 korrespondierend mit der Umdrehungszahl des Hülsenabschnitts 18 von dem Zapfen 32 betätigt wird. Mit anderen Worten entspricht die Anzahl der Umdrehungen beispielsweise einem Quotienten aus der Anzahl der Betätigungen des zweiten Schalters 42 und der Anzahl der Zapfen 32 (Rastelemente). Beispielsweise kann der Hülsenabschnitt 18 symmetrisch ausgebildet sein, d.h. zwei in Umfangsrichtung gegenüberliegende Zapfen 32 besitzen. Dann wird der zweite Schalter 42 je Umdrehung des Hülsenabschnitts 18 zweimal betätigt. So können auch halbe Umdrehungen des Hülsenabschnitts 18 erfasst werden. Durch mehrere Zapfen 32 ist gleichzeitig eine mechanische Verrastung gewährleistet. Dadurch kann eine Umdrehungszahl des Hülsenabschnitts 18 erfasst werden. Insbesondere ist der Hülsenabschnitt 18 so ausgebildet, dass er in der ersten Axialposition von dem Schneidabschnitt 4 drehentkoppelt und in der zweiten Axialposition mit dem Schneidabschnitt 4 drehgekoppelt ist.

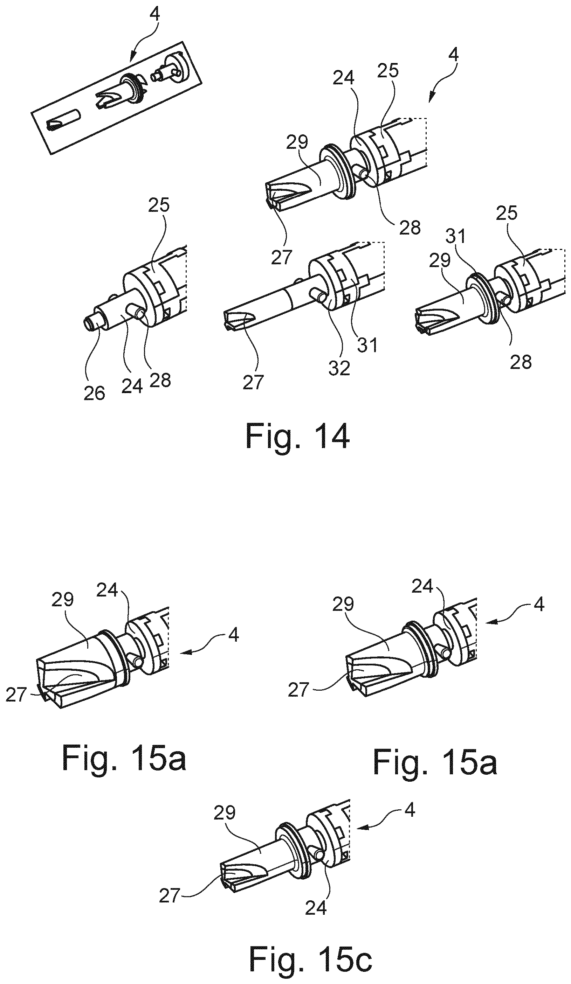

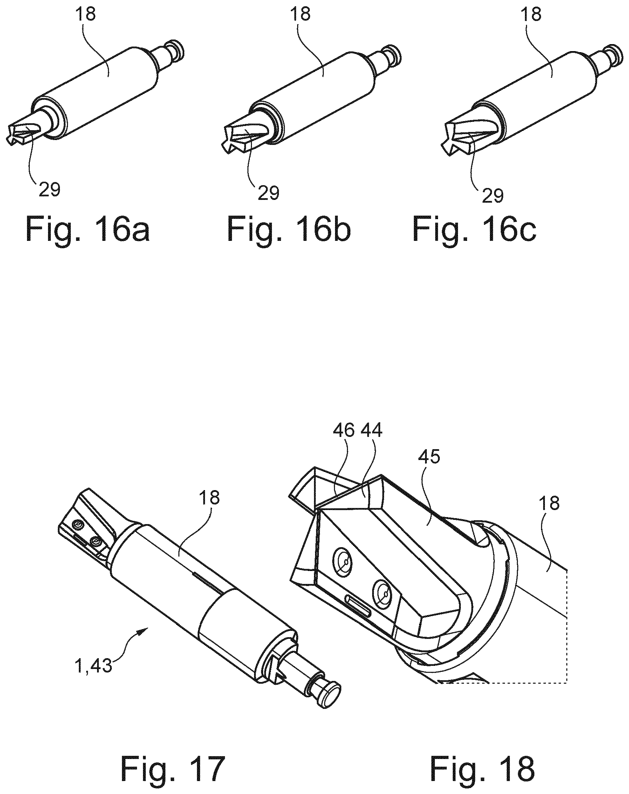

Fign. 14 bis 16 zeigen den Aufbau des Schneidabschnitts 4 gemäß einem weiteren Aspekt der Erfindung. Wie oben beschrieben, weist der Schneidabschnitt 4 den Basisabschnitt 24 auf, der über die erste Schnittstelle 26 mit dem ersten Eingriffsabschnitt 27 und über die zweite Schnittstelle 28 mit dem zweiten Eingriffsabschnitt 29 verbunden ist. Vorzugsweise sind der Basisabschnitt 24, der erste Eingriffsabschnitt 27 und der zweite Eingriffsabschnitt 29 als Metallbauteil ausgebildet. Der Basisabschnitt 24 dient als Kraftübertragungseinzelteil und ist für Schneidabschnitte 4 unterschiedlicher Größe gleichbleibend ausgebildet. Über die erste Schnittstelle 26 können unterschiedlich große erste Eingriffsabschnitte 27, d.h. Innenfräser, auf das Gewinde aufgeschraubt werden. Über die zweite Schnittstelle 28 können unterschiedlich große zweite Eingriffsabschnitt 29, d.h. Außenfräser, aufgeschoben werden (vgl. Fign. 15a bis 15c). Der Außendurchmesser des Flansches 31 des zweiten Eingriffsabschnitts 29 ist bei unterschiedlich großen zweiten Eingriffsabschnitten 29 gleichbleibend ausgebildet. Dadurch kann für Schneidabschnitte 4 unterschiedlicher Größe der gleiche Hülsenabschnitt 18 (vgl. Fign. 16a bis 16c) verwendet werden.

Fign. 17 und 18 zeigen eine weitere Ausführungsform gemäß einem weiteren Aspekt der Erfindung. Das Werkzeug 1 ist abgesehen von seinen Schneiden vollständig als Kunststoffbauteilwerkzeug 43 ausgebildet. Der erste Eingriffsabschnitt 27 ist als ein Kunststoffbauteil 44, wie ein Kunststoffdom, ausgebildet. Der zweite Eingriffsabschnitt 29 ist als ein Kunststoffbauteil 49, wie ein Kunststoffdom, ausgebildet. Ein Einlegebauteil 46 aus Metall ist mit dem ersten Eingriffsabschnitt 27 und/oder dem zweiten Eingriffsabschnitt 29 durch Warmprägen verbunden.

## Patentansprüche

1. Medizinisches Werkzeug (1), das als ein drehantreibbares Werkzeug, insbesondere als ein spanabhebendes Werkzeug, vorzugsweise als ein Trepanationswerkzeug, ausgebildet ist und das einen mit einer medizinischen Vorrichtung (2) zur insbesondere formschlüssigen Drehmomentübertragung verbindbaren Antriebsabschnitt (3) und einen mit dem Antriebsabschnitt (3) drehmomentübertragend koppelbaren Schneidabschnitt (4) hat, **dadurch gekennzeichnet, dass** das Werkzeug (1) eine elektronische Baugruppe (41) aufweist, die ausgebildet ist, um durch ein Entkoppeln des Schneidabschnitts (4) von dem Antriebsabschnitt (3) aktiviert zu werden.

2. Werkzeug (1) nach Anspruch 1, **dadurch gekennzeichnet, dass** die elektronische Baugruppe (41) einen vorzugsweise als einen Taster ausgebildeten Schalter (42) hat, der so von einem mit dem Schneidabschnitt (4) drehkoppelbaren Betätigungsabschnitt (32) mechanisch betätigbar ist, dass er die Anzahl der Umdrehungen des Schneidabschnitts (4), insbesondere nach dem Entkoppeln des Schneidabschnitts (4) von dem Antriebsabschnitt (3), erfasst.

3. Werkzeug (1) nach Anspruch 2, **dadurch gekennzeichnet, dass** der Betätigungsabschnitt (32) so angeordnet und ausgebildet ist, dass er mit dem Schneidabschnitt (4) drehgekoppelt ist, wenn der Schneidabschnitt (4) von dem Antriebsabschnitt (3) entkoppelt ist.

4. Werkzeug (1) nach einem der Ansprüche 1 bis 3, **dadurch gekennzeichnet, dass** die elektronische Baugruppe in einem feststehenden Bauteil des Werkzeugs (1) angeordnet ist.

5. Medizinisches Werkzeug (1) nach einem der Ansprüche 1 bis 4, **dadurch gekennzeichnet, dass** das Werkzeug (1) eine weitere elektronische Baugruppe (7) aufweist, die ausgebildet ist, um durch ein Verbinden des Werkzeugs (1) mit der medizinischen Vorrichtung (2), vorzugsweise durch einen Steckvorgang des Werkzeugs (1) in die medizinische Vorrichtung (2), aktiviert zu werden.

6. Werkzeug (1) nach Anspruch 5, **dadurch gekennzeichnet, dass** die weitere elektronische Baugruppe (7) eine Speichereinrichtung hat, in der werkzeugspezifische Daten gespeichert sind, die bei Aktivierung der elektronischen Baugruppe (7) vorzugsweise an eine externe Verarbeitungseinheit übermittelt werden.

7. Werkzeug (1) nach Anspruch 5 oder 6, **dadurch gekennzeichnet, dass** die weitere elektronische Baugruppe (7) einen weiteren Schalter (8, 9, 10) hat, der durch das Verbinden des Werkzeugs (1) mechanisch so betätigbar ist, dass der weitere Schalter (8, 9, 10) einen Stromkreis der elektronischen Baugruppe (7) in einer betätigten Schaltstellung schließt und in einer unbetätigten Schaltstellung öffnet.

8. Werkzeug (1) nach Anspruch 6 und 7, **dadurch gekennzeichnet, dass** die weitere elektronische Baugruppe eine Kommunikationsvorrichtung (11, 12, 15) zur Erzeugung einer Funkverbindung aufweist, die bei geschlossenem Stromkreis ein Funksignal mit den werkzeugspezifischen Daten überträgt und vorzugsweise in einem Kunststoffgehäuse (19) des Werkzeugs (1) angeordnet ist.

9. Werkzeug (1) nach Anspruch 7 oder 8, **dadurch gekennzeichnet, dass** der weitere Schalter (8, 9, 10) durch das Verbinden des Werkzeugs (1) in Axialrichtung oder in Radialrichtung zwischen der betätigten Schaltstellung und der unbetätigten Schaltstellung verlagerbar ist.

10. Werkzeug (1) nach einem der Ansprüche 5 bis 9, **dadurch gekennzeichnet, dass** die weitere elektronische Baugruppe (7) eine Rückmeldevorrichtung (34, 35) aufweist und/oder mit einer externen Rückmeldeeinrichtung verbindbar ist, und dass die Rückmeldevorrichtung (34, 35) und/oder die Rückmeldeeinrichtung so ausgebildet sind/ist, dass eine akustische und/oder visuelle Rückmeldung ausgegeben wird, wenn die elektronische Baugruppe (7) aktiviert wird oder aktiviert ist.

## Claims

1. The medical tool (1), which is in the form of a rotatably drivable tool, in particular a cutting tool, preferably a trepanation tool, having a drive portion (3) which can be connected to a medical device (2) for in particular interlockingly transmitting torque and a cutting portion (4) which can be coupled to the drive portion (3) so as to transmit torque, **characterised in that** the tool (1) comprises an electronic assembly (41) which is designed to be activated by the cutting portion (4) being decoupled from the drive portion (3).

2. The tool (1) according to claim 1, **characterised in that** the electronic assembly (41) has a switch (42) which is preferably in the form of a push button and which can, by an activating portion (32) which can be rotatably coupled to the cutting portion (4), be activated mechanically such that it acquires the number of revolutions of the cutting portion (4), in particular after the decoupling of the cutting portion (4) from the drive portion (3).

3. The tool (1) according to claim 2, **characterised in that** the activating portion (32) is arranged and designed such that it is rotatably coupled to the cutting portion (4) when the cutting portion (4) is decoupled from the drive portion (3).

4. The tool (1) according to one of claims 1 to 3, **characterised in that** the electronic assembly is arranged in a stationary component of the tool (1).

5. A medical tool (1) according to one of the claims 1 to 4, **characterised in that** the tool (1) comprises a further electronic assembly (7) which is designed to be activated by the tool (1) being connected to the medical device (2), preferably by an operation of plugging the tool (1) into the medical device (2).

6. The tool (1) according to claim 5, **characterised in that** the further electronic assembly (7) has a storage device storing tool-specific data which are transmitted preferably to an external processing unit during activation of the electronic assembly (7).

7. The tool (1) according to claims 5 or 6, **characterised in that** the further electronic assembly (7) has a further switch (8, 9, 10) which can be activated mechanically by the connecting of the tool (1) such that the further switch (8, 9, 10) closes an electric circuit of the electronic assembly (7) in an activated switching position and opens it in a non-activated switching position.

8. The tool (1) according to claims 6 and 7, **characterised in that** the further electronic assembly comprises a communication device (11, 12, 15) for generating a radio communication which transmits, in the case of a closed electric circuit, a radio signal with the tool-specific data and is preferably arranged in a plastic housing (19) of the tool (1).

9. The tool (1) according to claims 7 or 8, **characterised in that** the switch (8, 9, 10) is, by means of the connecting of the tool (1), displaceable in the axial direction or in the radial direction between the activated switching position and the non-activated switching position.

10. The tool (1) according to one of claims 5 to 9, **characterised in that** the further electronic assembly (7) comprises a feedback device (34, 35) and/or can be connected to an external feedback means, and that the feedback device (34, 35) and/or the feedback means is/are designed such that an acoustic and/or visual feedback is output when the electronic assembly (7) is activated or is being activated.

## Revendications

1. Outil médical (1) qui est conçu comme un outil pouvant être entraîné en rotation, en particulier comme un outil d'enlèvement de copeaux, de préférence comme un outil de trépanation, et qui présente une section d'entraînement (3) pouvant être connectée à un dispositif médical (2) pour la transmission de couple, en particulier par complémentarité de formes, et une section de coupe (4) pouvant être couplée à la section d'entraînement (3) par transmission de couple, **caractérisé en ce que** l'outil (1) présente un module électronique (41) qui est conçu pour être activé par un découplage de la section de coupe (4) de la section d'entraînement (3).

2. Outil (1) selon la revendication 1, **caractérisé en ce que** le module électronique (41) présente un commutateur (42), de préférence comme un bouton-poussoir, qui peut être actionné mécaniquement par une section d'actionnement (32) pouvant être couplée en rotation à la section de coupe (4) de sorte qu'il détecte le nombre de tours de la section de coupe (4), en particulier après le découplage de la section de coupe (4) de la section d'entraînement (3).

3. Outil (1) selon la revendication 2, **caractérisé en ce que** la section d'actionnement (32) est disposée et configurée de sorte qu'elle est couplée en rotation avec la section de coupe (4) lorsque la section de coupe (4) est découplée de la section d'entraînement (3).

4. Outil (1) selon l'une quelconque des revendications 1 à 3, **caractérisé en ce que** le module électronique est disposé dans un composant fixe de l'outil (1).

5. Outil médical (1) selon l'une quelconque des revendications 1 à 4, **caractérisé en ce que** l'outil (1) présente un module électronique supplémentaire (7) qui est conçu pour être activé par une connexion de l'outil (1) au dispositif médical (2), de préférence par une opération d'enfichage de l'outil (1) dans le dispositif médical (2).

6. Outil (1) selon la revendication 5, **caractérisé en ce que** le module électronique supplémentaire (7) présente un appareil de mémoire dans lequel des données spécifiques à l'outil sont enregistrées, données qui sont de préférence transmises à une unité de traitement externe lors de l'activation du module électronique (7).

7. Outil (1) selon la revendication 5 ou 6, **caractérisé en ce que** le module électronique supplémentaire (7) présente un commutateur supplémentaire (8, 9, 10) qui peut être actionné mécaniquement par la connexion de l'outil (1) de sorte que le commutateur supplémentaire (8, 9, 10) ferme un circuit électrique du module électronique (7) dans une position de commutation actionnée et l'ouvre dans une position de commutation non actionnée.

8. Outil (1) selon les revendications 6 et 7, **caractérisé en ce que** le module électronique supplémentaire présente un dispositif de communication (11, 12, 15) pour générer une liaison radio qui transmet un signal radio avec les données spécifiques à l'outil lorsque le circuit électrique est fermé et est de préférence disposé dans un boîtier en plastique (19) de l'outil (1).

9. Outil (1) selon la revendication 7 ou 8, **caractérisé en ce que** le commutateur supplémentaire (8, 9, 10) peut être déplacé en direction axiale ou en direction radiale entre la position de commutation actionnée et la position de commutation non actionnée par la connexion de l'outil (1).

10. Outil (1) selon l'une quelconque des revendications 5 à 9, **caractérisé en ce que** le module électronique supplémentaire (7) présente un dispositif de rétroaction (34, 35) et/ou peut être connecté à un appareil de rétroaction externe, et **en ce que** le dispositif de rétroaction (34, 35) et/ou l'appareil de rétroaction sont/est conçu(s) de sorte qu'une rétroaction acoustique et/ou visuelle est émise lorsque le module électronique (7) est en cours d'activation ou est activé.
